# EUROPEAN PATENT APPLICATION

(11) **EP 4 649 925 A1**
(43) Date of publication of application: **19.11.2025**
(21) Application number: 24175871.3
(22) Date of filing: 15.05.2024
(51) Int. Cl.: A61F 5/01, A61F 5/058, A61F 5/10

(54) **THUMB ORTHOSIS**

(71) Applicant: WE Design B.V., 6814 EN Arnhem (NL)
(72) Inventor: Groen, Robert Gregor, 7396 NH Terwolde (NL)
(74) Representative: Nederlandsch Octrooibureau

(57) **Abstract**

A thumb orthosis (1) for support of a patient's thumb (T), comprising a thumb wire section (2) to receive a first metacarpal portion (M1) of the thumb; a dorsal section (3) comprising a radial end (3a) and an opposing ulnar end (3b), the radial end connects to the thumb wire section. The dorsal section is configured to extend in a lateral direction (LD) along the dorsal side of the hand. An ulnar wire section (4) connects to the ulnar end and is configured to extend along the dorsal side and around the ulnar side of the hand. The ulnar wire section comprises a hypothenar end (4b) to engage the hypothenar region (R) of the hand, and wherein the dorsal section has a width (X) equal to or larger than a width (Y) of the ulnar wire section in a proximal-distal direction (PD).

## Description

### Field of the invention

The present invention relates to a thumb orthosis, in particular a wire frame thumb orthosis offering support of the thumb in case of osteoarthritis, rheumatoid arthritis, adduction of CMC1 joint, CMC1 joint capsule and/or instability problems.

### Background art

Dutch patent publication NL 2016047 B1 discloses an orthosis for immobilizing a carpometacarpal joint of a thumb of a hand, comprising a joint supporting part and a holding part, wherein the joint supporting part comprises at least one anatomically shaped rigid ring-shaped element configured to be slid onto the thumb to enclose part of a thenar eminence of the thumb. The holding part comprises a rigid stabilizing part that is fixed at a first position to the joint supporting part and is configured to reach sideways along a first side of the hand, e.g. the palmar side, to engage a pinky side of the hand. The holding part further comprises an adjustable stabilizing part, such as an adjustable strap, configured to reach along a second side of the hand, e.g. the dorsal side, to connect a second position of the joint supporting part to a distal end of the rigid stabilizing part.

Prior art thumb orthoses or braces of the type mentioned above, and in particular the rigid stabilizing part extending along the palmar side of the hand, often impose particular restrictions of hand movements reducing a patient's ability to firmly grab objects with the hand.

### Summary

It is an object of the present invention to provide an improved thumb orthosis for support of the thumb, particularly for correcting adduction of the CMC1 joint, CMC1 and/or MCP1 joint capsule problems and joint instability problems whilst offering improved comfort and relief of the palmar region of the hand, thereby improving mobility for grabbing objects.

According to the present invention, a thumb orthosis of the type mentioned above is provided, comprising a ring shaped thumb wire section configured to receive a first metacarpal portion of the thumb; a dorsal section comprising a radial end and an opposing ulnar end, wherein the radial end is connected to the thumb wire section, and wherein the dorsal section is configured to extend in a lateral direction along the dorsal side of the hand. An ulnar wire section is provided and comprises a dorsal end connected to the ulnar end of the dorsal section and configured to extend in the lateral direction along the dorsal side and around the ulnar side of the hand, and wherein the ulnar wire section comprises a hypothenar end configured to engage the hypothenar region of the hand; and wherein dorsal section has a width equal to or larger than a width of the ulnar wire section in a proximal-distal direction.

The dorsal section and ulnar wire section provide all the stability needed such that the thumb wire section maintains the first metacarpal portion of a patient's thumb in a required position. By utilizing the dorsal section and ulnar wire section eliminates the need for a palmar wire section, thereby allowing a patient to grab objects in a manner that feels natural as an unobstructed palmar region of the hand provides more tactile feedback when engaging objects. Further, this design allows for a dorsal and ulnar pulling effect on the thumb which creates improved abduction compared to a palmar push when a palmar wire section is used instead. Surface pressure on the dorsal side of the hand is distributed by virtue of the dorsal section having a width equal to or larger than the width of the ulnar wire section.

### Short description of drawings

The present invention will be discussed in more detail below, with reference to the attached drawings, in which
Figure 1 shows a three-dimensional view of a thumb orthosis according to an embodiment of the present invention;
Figure 2 shows a three-dimensional view of a thumb orthosis according to another embodiment of the present invention;
Figure 3 shows a top view of a thumb orthosis according to an embodiment of the present invention;
Figure 4 shows a three-dimensional view a thumb orthosis according to yet another embodiment of the present invention;
Figure 5 shows a radial view of a thumb orthosis fitted to a patient's hand according to an embodiment of the present invention;
Figure 6 shows a palmar view of a thumb orthosis fitted to a patient's hand according to an embodiment of the present invention;
Figure 7 shows a dorsal view of a thumb orthosis fitted to a patient's hand according to an embodiment of the present invention.

### Detailed description of embodiments

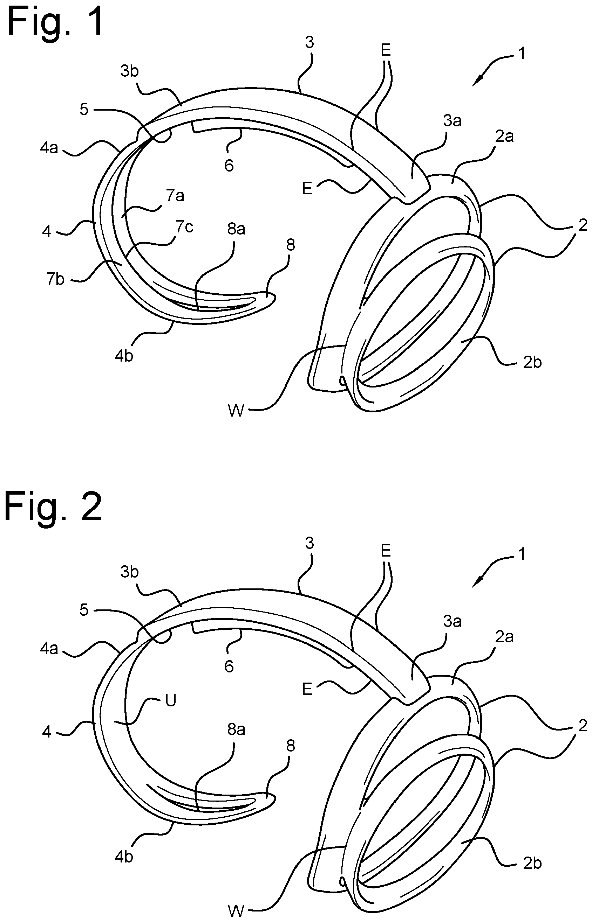

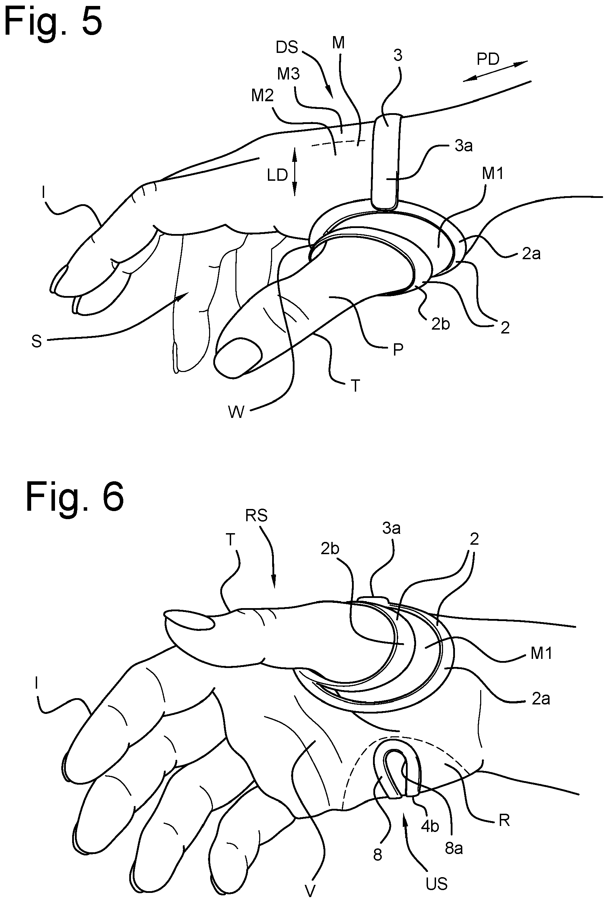

Referring to Figures 1, 3, 5 and 6, a thumb orthosis 1 according to an embodiment of the present invention is depicted, wherein Figure 5 shows how the thumb orthosis 1 is fitted to a patient's hand and supporting the patient's thumb T.

In particular, the thumb orthosis 1 comprises a ring shaped thumb wire section 2 which is configured to receive a first metacarpal portion M1 of the thumb T. A dorsal section 3 is provided and comprises a radial end 3a and an opposing ulnar end 3b, wherein the radial end 3a is connected to the thumb wire section 2. As shown, the dorsal section 3 is configured to extend in a lateral direction LD along the dorsal side DS of the hand, and in particular along a metacarpal region M thereof.

An ulnar wire section 4 is provided and comprises a dorsal end 4a connected to the ulnar end 3b of the dorsal section 3, wherein the ulnar wire section 4 is configured to extend in the lateral direction LD along the dorsal side DS and around the ulnar side US of the hand. The ulnar wire section 4 further comprises a hypothenar end 4b which is configured to engage the hypothenar region R of the hand.

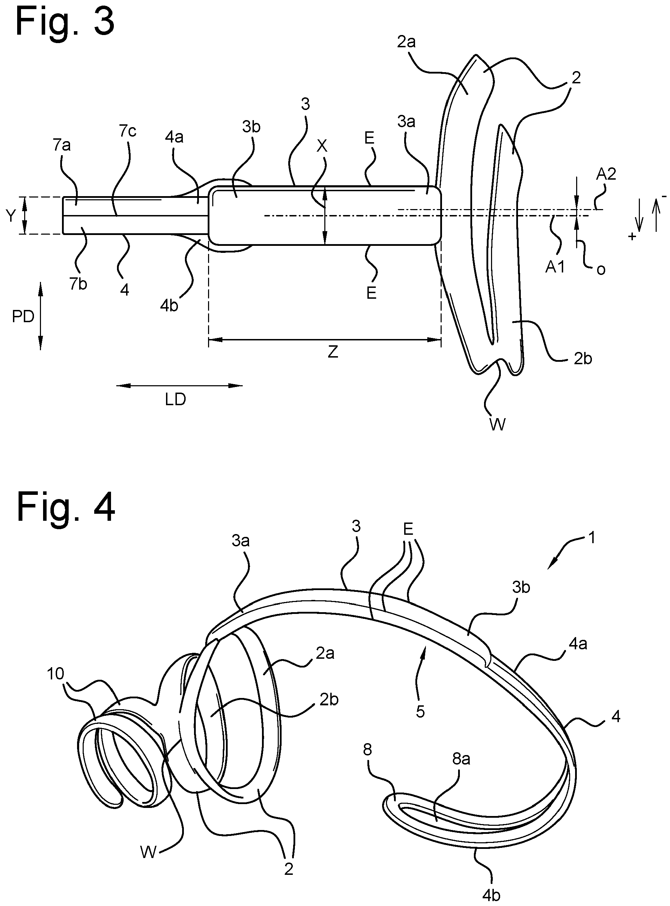

In Figure 3 it is shown that the dorsal section 3 has a width X which is equal to or larger than a width Y of the ulnar wire section 4 in a proximal-distal direction PD.

Note that in the exemplary embodiment shown in Figure 3, the dorsal section 3 and the ulnar wire section 4 may extend substantially parallel in the lateral direction LD, e.g. in a substantially straight line, and need not exhibit curvature in the proximal-distal direction PD.

According to the invention, the dorsal section 3 and ulnar wire section 4 provide all the stability needed with respect to the hand such that the thumb wire section 2 maintains the first metacarpal portion M1 of a patient's thumb T in a required position in case of osteoarthritis, rheumatoid arthritis, adduction of CMC1 joint, CMC1 joint capsule and/or instability problems.

By utilizing the dorsal section 3 and ulnar wire section 4 eliminates the need for providing stability by means of a palmar wire section extending along a palmar region V of the hand as shown in Figure 6. By leaving the palmar region V free allows a patient to grab objects in a manner that feels natural as the palmar region V provides more tactile feedback when engaging objects. Further, this design allows for a pulling effect on the thumb T from the dorsal side DS and the ulnar side US, which creates improved abduction as compared to a push from the palmar region V. Surface pressure on the dorsal side DS of the hand, in particular along the metacarpal region M thereof, is distributed by virtue of the dorsal section 3 having a width X equal to or larger than the width Y of the ulnar wire section 4.

In an advantageous embodiment, the width X of the dorsal section 3 is larger than the width Y of the ulnar wire section 4, thereby further distributing surface pressure along the dorsal side DS of the hand. In a group of exemplary embodiments, the width X of the dorsal section 3 and the width Y of the ulnar wire section 4 satisfy the inequality such as X > 1.1 * Y or X > 1.2 * Y or X > 1.3 * Y of X > 1.4 * Y or X > 1.5 * Y. By increasing the width X of the dorsal section 3 with respect to the width Y of the ulnar wire section 4 increases overall stability of the thumb orthosis 1.

It will be understood that the thumb orthosis 1 of the present invention will be tailor made and specifically sized and shaped to snugly fit to a specific hand of a patient. For example, to achieve the required size and shape, the thumb wire section 2, the dorsal section 3, and the ulnar wire section 4 will be cut at length, bend to a specific shape, and subsequently connected. For further changes, the thumb orthosis 1 may also be bent when fully assembled.

In an embodiment the thumb wire section 2, the dorsal section 3, and the ulnar wire section 4 are made of a metallic material and connected by means of a welding process. In an advantageous embodiment the metallic material is silver for its antibacterial properties.

Instead of bending the thumb wire section 2, the dorsal section 3 and the ulnar wire section 4 into shape, an alternative embodiment is conceivable wherein the thumb wire section 2, the dorsal section 3, and the ulnar wire section 4 form a single unitary piece, e.g. are integrally formed by means of a casting process. Note that a thumb orthosis 1 obtained by a casting process may be used as a master model from which further castings of the thumb orthosis 1 can be made, thereby achieving increased consistency between thumb orthoses as well as speeding up the manufacturing process and lowering cost. Furthermore, it is even conceivable that particular parts of the thumb orthosis 1 are cast and act as a master model from which further castings are made. For example, it is possible to only cast the thumb wire section 2, the dorsal section 3 and/or the ulnar wire section 4, which may then be used as a master model to produce further castings of the thumb wire section 2, the dorsal section 3 and/or the ulnar wire section 4.

In an embodiment, the dorsal section 3 has a length Z in the lateral direction LD such that the dorsal section 3 is configured to extend at least across a second metacarpal portion M2 and a third metacarpal portion M3 of the hand. By spanning the second and third metacarpal portions M2, M3 ensures that, in conjunction with the aforementioned width X of the dorsal section 3, surface pressure on the dorsal side DS of the hand is distributed for avoiding discomfort whilst increasing stable support offered by the thumb wire section 2.

From Figure 3 it can be seen that in an embodiment the length Z of the dorsal section 3 is larger than the width X thereof. This provides an elongated dorsal section 3 able to sufficiently extend along the metacarpal region M on the dorsal side DS of the hand for facilitating stability of the thumb orthosis 1. In a specific embodiment as depicted, the dorsal section 3 is a rectangular piece or part for providing sufficient distribution of surface pressure on the dorsal side DS of the hand. This rectangular piece or part may be seen as a substantially flat plate for engaging the dorsal side DS.

To prevent discomfort, the thumb orthosis 1 should engage the hand in congruent manner such that contours of the hand are followed and pressure points are avoided. Figure 1 depicts an embodiment wherein the dorsal section 3 is an arched unitary piece or part that comprises a concave inner side 5 for congruent engagement with the dorsal side DS of the hand. In particular, the arched unitary piece is able to extend along the dorsal side DS of the hand such that the concave inner side 5 snugly follows the dorsal side for good surface engagement and pressure distribution, thereby avoiding pressure points that may cause discomfort.

Comfort can be further improved by an embodiment wherein the concave inner side 5 is provided with a flexible pressure pad 6. This flexible pressure pad 6 absorbs small irregularities of the dorsal side DS of the hand such that the smallest of pressure points can be avoided. Also, the flexible pressure pad 6 maintains snug engagement in case curvature of the dorsal side DS slightly changes when an object is grabbed with the hand. As the dorsal side DS of the hand has very little soft tissue compared to the palmar region V of the hand, the dorsal section 3 may be provided with one or more rounded edges E to further prevent pressure points by removing sharpness of edges of the dorsal section 3. In case the dorsal section 3 is a rectangular piece as depicted in Figure 3, for example, then all peripheral edges E may be rounded or chamfered to improve comfort.

In Figure 1 and 3 there is depicted an embodiment wherein the ulnar wire section 4 is a single unitary piece comprising two parallel wire strands 7a, 7b connected to the ulnar end 3b of the dorsal section 3 and forming a continuous U-shaped loop 8 at the hypothenar end 4b. The two parallel wires strands 7a, 7b provide a larger inner surface for engagement with the dorsal side DS and ulnar side US of the hand for pressure distribution. By using a U-shaped loop 8 at the hypothenar end 4b allows for a single stretch of wire. That is, the single stretch of wire can be bent to form the continuous U-shaped 8 at the hypothenar end 4b and to obtain the two parallel wire strands 7a, 7b.

The single stretch of wire for forming the ulnar wire section 4 may have an oval cross section to avoid sharp edges that could cause discomfort. The oval cross section also provides a sufficiently flat surface for engagement with the dorsal side DS and ulnar side US of the hand. Furthermore, the single stretch of wire may be made of a metallic, e.g. silver, material that can be readily bent into shape and maintain this shape with sufficient rigidity.

Note that in an embodiment the continuous U-shaped loop 8 may enclosed an aperture or opening 8a, wherein the loop 8 can be sized to achieve a required surface area for support against the hypothenar region R, see e.g. Figure 6.

As exemplified in Figure 1 and 3, in an embodiment the two parallel wire strands 7a, 7b are connected along the lateral direction LD between the dorsal end 4a of the ulnar wire section 4 and the hypothenar end 4b thereof. In this embodiment the two parallel wire strands 7a, 7b are connected along a seam 7c which extends in the lateral direction LD, wherein the connection along the seam 7c provides strength and improved stability to the thumb orthosis 1.

Note that the two parallel wire strands 7a, 7b may diverge when approaching the hypothenar end 4b for forming the continuous U-shaped loop 8 enclosing the aperture/opening 8a.

In Figure 2 there is shown an embodiment wherein the ulnar wire section 4 comprises a smooth continuous inner surface U for engagement with the ulnar side US and dorsal side DS of the hand. In this embodiment the ulnar wire section 4 exhibits no inner surface irregularities that could cause discomfort along the dorsal side DS and/or ulnar side US of the hand. For example, when the ulnar wire section 4 comprises the two parallel wire strands 7a, 7b connected along the seam 7c, then the seam 7c may cause discomfort where it engages the ulnar side US and/or dorsal side DS of the hand. Also, surface irregularities such as the seam 7c may cause hygiene problems due to accumulation of dirt, bacteria etc. overtime. Therefore, removing the seam 7c along the inner side of the ulnar wire section 4 to obtain the smooth continuous inner surface U increases comfort as well as hygiene.

In one embodiment the seam 7c may be filled with a welding material and spread out to obtain the smooth continuous inner surface U. In another embodiment the two parallel wires strands 7a, 7b may be connected along the seam 7c and sufficiently fused together to obtain the smooth continuous inner surface U. In an embodiment wherein the ulnar wire section 4 is made of a metallic material, e.g. silver, then the smooth continuous inner surface U can be obtained by fusing/welding the two parallel wire strands 7a, 7b. Further welding material may be added to remove any surface irregularities when needed.

As mentioned earlier, the ring shaped thumb wire section 2 is configured to receive the first metacarpal portion M1 of the thumb T. As shown, an embodiment is provided wherein the thumb wire section 2 comprises two spaced apart rings 2a, 2b for receiving the first metacarpal portion M1, wherein the use of these two rings provides distributed support to the thumb T, thereby improving comfort and preventing pressure points while maintaining an open structure.

In a particular embodiment, the thumb wire section 2 may comprise two spaced apart oval shaped ring portions 2a, 2b for receiving the first metacarpal portion M1 of the thumb T, and wherein the two ring portions 2a, 2b locally connect at a web point W configured to be received by a first interdigital space S between the thumb T and index finger I. By connecting the two ring portions 2a, 2b at the web point W, such that this web point W is received by the first interdigital space S, ensures that maximal mobility of the thumb T is maintained whilst properly supporting the first metacarpal portion M1.

From Figures 1, 3, 5 and 6 it can be seen that an embodiment is provided wherein the two oval shaped ring portions 2a, 2b may comprise an oval shaped proximal ring portion 2a and an oval shaped distal ring portion 2b. The proximal ring portion 2a has a first circumferential size, and the distal ring portion 2b has a second circumferential size, wherein the first circumferential size is lager than the second circumferential size. The difference between the first and second circumferential sizes takes tapering of the thumb T into account such that both the proximal and distal ring portions 2a, 2b are able to maintain a snug and supportive fit with the first metacarpal portion M1.

In a further embodiment, the radial end 3a of the dorsal section 3 is connected to the proximal ring portion 2a. In this embodiment the connection between the proximal ring portion 2a and the dorsal section 3 is sufficient to achieve structural rigidity and stability of the thumb orthosis 1. The connection between the distal ring portion 2b and the proximal ring portion 2a is achieved by virtue of the web point W as mentioned earlier.

Like the ulnar wire section 4, an embodiment is conceivable wherein the proximal ring portion 2a and the distal ring portion 2b are made of wire material which is bent for forming an oval shaped ring. The wire material may be metallic, e.g. silver, and may have an oval cross section for avoiding sharp edges that could cause discomfort. The oval cross section provides a sufficiently flat surface for engagement with the first metacarpal portion M1 of the thumb T.

As suggested earlier, instead of bending wire material, it is conceivable that the proximal ring portion 2a and the distal ring portion 2b are integrally formed by a casting process. The resulting thumb wire section 2 may then act as a master model for further castings of the proximal ring portion 2a and the distal ring portion 2b.

Various connections may be utilized between the dorsal section 3 and the thumb wire section 2. For example, Figure 3 shows that the dorsal section 3 may comprise a lengthwise central axis A1 in the lateral direction LD, and the proximal ring portion 2a may comprise an axial central axis A2 in the lateral direction LD. To further optimize the thumb orthosis 1, the lengthwise central axis A1 and the axial central axis A2 may be offset, i.e. offset by a predetermined offset distance "o". The offset distance o between axes A1 and A2 may be chosen to achieve a required fit of the thumb orthosis 1.

For example, in an embodiment the offset distance o may be positive (+) as depicted in Figure 3. That is, the lengthwise central axis A1 of the dorsal section 3 may be offset in a positive direction (+) with respect to the axial central axis A2 of the first proximal ring portion 2a. Note that in correspondence with the orientation of the thumb orthosis 1 as shown in Figure 3 and 5, the positive direction (+) may be considered a distal direction, so that the lengthwise central axis A1 may be offset in distal direction with respect to the axial central axis A2.

In another embodiment, not shown, the offset distance o may be negative (-) as depicted in Figure 3, wherein the lengthwise central axis A1 of the dorsal section 3 may be offset in a negative direction (-) with respect to the axial central axis A2 of the first proximal ring portion 2a. In correspondence with the orientation of the thumb orthosis 1 as shown in Figure 3 and 5, the negative direction (-) may be considered a proximal direction, so that the lengthwise central axis A1 may be offset in proximal direction with respect to the axial central axis A2.

In yet another embodiment, not shown, the offset distance o may be zero, i.e. the lengthwise central axis A1 aligns with the axial central axis A2 and as such the axes A1, A2 coincide.

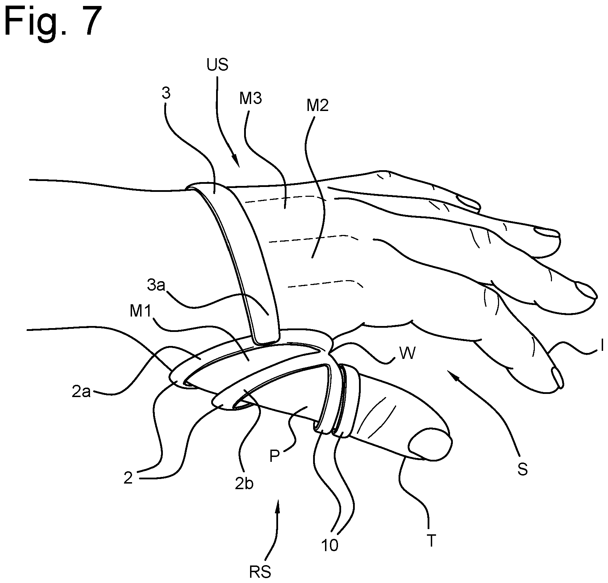

Referring to Figure 4 and 7, according to the present invention the thumb orthosis 1 can be utilized for supporting the first metacarpal portion M1 of the thumb T but also for supporting the proximal phalange portion P of the thumb T. In particular, an embodiment is provided wherein the thumb orthosis 1 further comprises a ring shaped auxiliary thumb wire section 10, which is spaced apart from the thumb wire section 2 and configured to receive the proximal phalange portion P of the thumb T. From the figures it can be readily seen that the auxiliary thumb wire section 10 locally connects to the thumb wire section 2. The ring shaped auxiliary thumb wire section 10 significantly immobilises the proximal phalange portion P of the thumb T for relieving the MCP1 joint, for example. In this way the thumb orthosis 1 provides support and stability to both the first metacarpal portion M1 as well as the proximal phalange portion P.

The auxiliary thumb wire section 10 may locally connect to the web point W at which the proximal ring portion 2a and the distal ring portion 2b are connected as well. Note that the web portion W will be located near the MCP1 joint when the thumb orthosis 1 is fitted to a patient's hand, so that the ring shaped auxiliary thumb wire section 10 can be readily configured to extend away from the web portion W for receiving the proximal phalange portion P of the thumb T.

In a further embodiment, the auxiliary thumb wire section 10 may be made of a single stretch of wire and formed as a ring shaped spiral section through which the proximal phalange portion P extends for support. A spiral shaped auxiliary thumb wire section 10 provides distributed support along he the proximal phalange portion P to avoid pressure points and discomfort.

Like the ulnar wire section 4, the wire material used for the auxiliary thumb wire section 10 may be metallic, e.g. silver, and may have an oval cross section for avoiding sharp edges that could cause discomfort. As mentioned earlier, the oval cross section provides sufficient surface area for pressure distribution along the proximal phalange portion P of the thumb T.

The present invention embodiments have been described above with reference to a number of exemplary embodiments as shown in and described with reference to the drawings. Modifications and alternative implementations of some parts or elements are possible, and are included in the scope of protection as defined in the appended claims.

## Claims

1. A thumb orthosis (1) for support of a patient's thumb (T), comprising a ring shaped thumb wire section (2) configured to receive a first metacarpal portion (M1) of the thumb (T);
a dorsal section (3) comprising a radial end (3a) and an opposing ulnar end (3b), wherein the radial end (3a) is connected to the thumb wire section (2), and wherein the dorsal section (3) is configured to extend in a lateral direction (LD) along the dorsal side of the hand;
an ulnar wire section (4) comprises a dorsal end (4a) connected to the ulnar end (3b) of the dorsal section (3) and configured to extend in the lateral direction along the dorsal side and around the ulnar side of the hand, and wherein the ulnar wire section (4) comprises a hypothenar end (4b) configured to engage the hypothenar region (R) of the hand; and
wherein dorsal section (3) has a width (X) equal to or larger than a width (Y) of the ulnar wire section (4) in a proximal-distal direction (PD).

2. The thumb orthosis (1) according to claim 1, wherein the dorsal section (3) has a length (Z) in the lateral direction (LD) configured to extend at least across the second and the third metacarpal portions (M2, M3) of the hand.

3. The thumb orthosis (1) according to claim 2, wherein the length (Z) of the dorsal section (3) is larger than the width (X) thereof.

4. The thumb orthosis (1) according to any of claims 1-3, wherein the dorsal section (3) is a rectangular piece.

5. The thumb orthosis (1) according to any of claims 1-4, wherein the dorsal section (3) is an arched unitary piece comprising a concave inner side (5) for congruent engagement with the dorsal side of the hand.

6. The thumb orthosis (1) according to claim 5, wherein the concave inner side (5) is provided with a flexible pressure pad (6).

7. The thumb orthosis (1) according to any of claims 1-6, wherein the ulnar wire section (4) is a single unitary piece comprising two parallel wire strands (7a, 7b) connected to the ulnar end (3b) of the dorsal section (3) and forming a continuous U-shaped loop (8) at the hypothenar end (4b).

8. The thumb orthosis (1) according to claim 7, wherein the two parallel wire strands (7a, 7b) are connected along the lateral direction (LD) between the dorsal end (4a) of the ulnar wire section (4) and the hypothenar end (4b) thereof.

9. The thumb orthosis (1) according to any one of claims 1-8, wherein the ulnar wire section (4) comprises a smooth continuous inner surface (U) for engagement with the dorsal side and ulnar side of the hand.

10. The thumb orthosis (1) according to any of claims 1-9, wherein the thumb wire section (2) comprises two spaced apart oval shaped ring portions (2a, 2b) for receiving the first metacarpal portion (M1) of the thumb (T), wherein the two ring portions (2a, 2b) locally connect at a web point (W) configured to be received by a first interdigital space (S) between the thumb (T) and index finger (I).

11. The thumb orthosis (1) according to claim 10, wherein an oval shaped proximal ring portion (2a) of the two ring portions (2a, 2b) has a first circumferential size, and wherein an oval shaped distal ring portion (2b) of the two ring portions (2a, 2b) has a second circumferential size, wherein the first circumferential size is larger than the second circumferential size.

12. The thumb orthosis (1) according to claim 11, wherein the radial end (3a) of the dorsal section (3) is connected to the proximal ring portion (2a).

13. The thumb orthosis (1) according to claim 12, wherein the dorsal section (3) has a lengthwise central axis (A1) in the lateral direction (LD), and wherein the proximal ring portion (2a) has an axial central axis (A2) in the lateral direction (LD), wherein the lengthwise central axis (A1) and the axial central axis (A2) are offset.

14. The thumb orthosis (1) according to any of claims 1-13, further comprising a ring shaped auxiliary thumb wire section (10) spaced apart from the thumb wire section (2) and configured to receive a proximal phalange portion (P) of the thumb (T), wherein the auxiliary thumb wire section (10) locally connects to the thumb wire section (2).

15. The thumb orthosis (1) according to claim 10 and 14 wherein the auxiliary thumb wire section (10) locally connects to the web point (W).
